# EUROPÄISCHE PATENTSCHRIFT

(11) **EP 2 390 391 B1**
(45) Veröffentlichungstag und Bekanntmachung des Hinweises auf die Patenterteilung: **19.12.2012**
(21) Anmeldenummer: 11170772.5
(22) Anmeldetag: 02.04.2003
(51) Int. Cl.: D01H 5/32, D01H 5/38, D01H 5/42, G01N 22/00

(54) **Spinnereivorbereitungsmaschine**
Spinning preparation machine
Machine de préparation de filature

(30) Priorität: 04.04.2002 DE 10214955
(43) Veröffentlichungstag der Anmeldung: 30.11.2011
(62) Teilanmeldung aus: 03712127.4
(73) Patentinhaber: Rieter Ingolstadt GmbH, 85055 Ingolstadt (DE)
(72) Erfinder: Dämmig, Joachim, 85053 Ingolstadt (DE); Ueding, Michael, 85049 Ingolstadt (DE); Cherif, Chokri, 01219 Dresden (DE)
(74) Vertreter: Schlief, Thomas P.

(56) Entgegenhaltungen:
- WO-A1-00/12974
- WO-A1-91/12518

## Beschreibung

Die Erfindung betrifft eine Spinnereivorbereitungsmaschine sowie ein Verfahren zur Kalibrierung einer Spinnereivorbereitungsmaschine.

In der Spinnereiindustrie wird beispielsweise aus Baumwolle in mehreren Prozessschritten zuerst ein vergleichmäßigter Faserverband und schließlich als Endprodukt ein gedrehtes Garn produziert. Die der Garnherstellung vorgeordneten Spinnereivorbereitungsmaschinen, wie Karden, Kämmmaschinen und Strecken, haben insbesondere die Aufgabe, die Bandmasseschwankungen eines oder mehrerer Faserbänder auszuregulieren. Zu diesem Zweck sind beispielsweise an Strecken Bandsensoren angeordnet, welche die Banddicke bzw. den Bandquerschnitt bzw. die Bandmasse bzw. deren Schwankungen messen und diese Informationen an eine Reguliereinheit weitergeben, die mindestens eines der Verzugsorgane des Streckwerks entsprechend ansteuert. Eine nach einem solchen Regulierprinzip arbeitende Strecke ist beispielsweise das Model RSB-D 30 der Firma RIETER. Auch bei unregulierten Strecken sind Informationen hinsichtlich der Banddickeschwankungen in vielen Fällen erwünscht. Ein entsprechender Sensor am Auslauf einer solchen Strecke gibt beispielsweise ein entsprechendes Abschaltsignal für die Maschine und/oder ein Warnsignal aus, wenn ein Schwellenwert der Bandmasse bzw. der Banddicke unter- bzw. überschritten wird.

Zur Messung der Banddickenschwankungen sind insbesondere mechanische Abtastungen bekannt, die sich heutzutage in fast allen entsprechenden Maschinen durchgesetzt haben. Allerdings reicht die Dynamik dieser mechanischen Sensoren bei Liefergeschwindigkeiten von mehr als 1000 m/min bei einem hohen Anforderungsprofil nicht mehr in genügendem Maße aus. Zudem macht sich die notwendige starke mechanische Verdichtung vor dem mechanischen Sensor negativ auf die Verzugsfähigkeit bemerkbar.

Neben der mechanischen Abtastung der Banddickenschwankungen sind weitere Abtastprinzipien vorgeschlagen worden. So ist beispielsweise aus der US 2,942,303 sowie der DE 44 45 720 A1 bekannt, die Banddicke berührungslos mit durchdringender optischer Strahlung zu messen. Jedoch wird die Messgenauigkeit hierbei stark von den Umgebungseinflüssen, z. B. Temperatur, Feuchtigkeit und Schmutz, beeinflusst. Außerdem ist das Verfahren anfällig gegenüber Farbe sowie Reflektionseigenschaften des Faserverbands.

Bei weiteren bekannten berührungslos arbeitenden Messverfahren werden Ultraschallwellen verwendet. Ebenfalls bekannt sind kapazitiv oder pneumatisch arbeitende Meßmethoden. Auch ist vorgeschlagen worden, Röntgenstrahlung oder gamma-Strahlen zu verwenden. Allen diesen Verfahren ist jedoch gemeinsam, dass sie feuchteempfindlich sind. Es nützt daher wenig, dass klimatische Einflüsse wie die Temperatur und die relative Luftfeuchtigkeit sich in der Regel kompensieren lassen, um klimatische Einflüsse minimieren zu können. Das Problem der inhärenten Faserfeuchte lässt sich hierdurch nicht ohne weiteres beseitigen. Zudem kann bei ein- und derselben Baumwollpartie die Faserfeuchte bei gleichbleibenden Umgebungsbedingungen bis zu 5 % variieren. Auch nehmen die oberen Baumwolllagen in einer Kanne, die einer Spinnereivorbereitungsmaschine zugestellt wird, mehr Feuchtigkeit auf als die darunter liegenden. Außerdem weisen die textilen Fasern durch die Veränderung der klimatischen Bedingungen innerhalb einer Spinnerei ― z. B. morgens vs. mittags vs. abends ― unterschiedliche Feuchte auf. Die genannten Einflüsse haben ihrerseits einen großen Einfluss auf das Messergebnis der Banddicke und somit auf die Regulierungsgüte. Insgesamt sind diese Verfahren also kaum für hochpräzise Messungen der Faserbanddicke geeignet.

Eine relativ neue Methode zur Messung der Banddicke beruht auf der Verwendung von Mikrowellen. In der WO 00/12974 ist ein derartiges Messverfahren unter Verwendung von Mikrowellen beschrieben, bei der Mikrowellen in einen Resonator eingekoppelt werden, durch die ein oder mehrere Faserbänder geführt werden. Es wird dann die Dämpfung und die Resonanzfrequenzverschiebung aufgrund der Anwesenheit des oder der Faserbänder gemessen und aus den Messwerten auf die Dickenschwankungen und evtl. den Feuchtegehalt des oder der Faserbänder geschlossen. In der EP 0 468 023 B1 ist ein ähnliches Mikrowellen-Messverfahren beschrieben, das sich auf die Messung von Faserbändern übertragen lässt. Die auf der Mikrowellen-Resonatortechnik beruhenden Sensoren bieten insbesondere den Vorteil, dass die Umweltbedingungen - wie beispielsweise die Raumtemperatur und die Raumfeuchte - schon mitberücksichtigt sind, so dass diese nicht weiter kompensiert werden müssen.

Allerdings sind die in den genannten Druckschriften beschriebenen und dargestellten Sensoren sowie die entsprechenden Messverfahren in vielfacher Hinsicht unausgereift und verbesserungswürdig. Insbesondere die spezifische Anpassung an die Problematik bei der Vermessung von Faserbändern erfordert neue Lösungen.

Es ist Aufgabe der Erfindung, die Genauigkeit bei der Vermessung von Faserbändern bzw. eines Faserverbands mittels Mikrowellen zu verbessern.

Diese Aufgabe wird bei einer Spinnereivorbereitungsmaschine gemäß Anspruch 1 und bei einem Verfahren gemäß den Ansprüchen 9 und 10 gelöst.

Gemäß der Erfindung werden zur Kalibrierung des mindestens einen Mikrowellensensors bevorzugt Kalibrierungskurven für unterschiedliche Materialien erstellt, wobei diese Kurven vorzugsweise in der Messelektronik speicherbar sind und/oder ein Abrufen der Kurven bei Bedarf von externen elektronischen Medien erfolgen kann, z. B. über das Internet, eine Compact Disc etc.

Für jedes Fasermaterial, z. B. Baumwolle, Polyester, Viskose, Polyacrylnitril etc, wird bevorzugt mindestens eine Kalibrierungskurve erstellt. Insbesondere in Abhängigkeit von beispielsweise Kräuselungsgrad, Feuchteaufnahmevermögen, Vorbehandlungsgrad, Verschmutzungsgrad etc. können auch vorteilhafterweise mehrere Kalibrierungskurven aufgenommen werden.

Wenn Fasermischungen vorliegen - z. B. Flockenmischungen und/oder Bandmischungen - sind neue Kalibrierungskurven in Abhängigkeit vom Mischungsverhältnis zu bestimmen. Diese Kurven können z. B. in einem elektronischen Speicher hinterlegt sein oder aufgrund einer Eingabe des Mischungsverhältnisses aus den entsprechenden einzelnen Kalibrierungskurven berechnet bzw. bestimmt werden. Hierzu kommen insbesondere mathematische Operationen in Frage, z. B. Mittelungen, Interpolationen oder Regressionen. Alternativ oder zusätzlich sind diese Daten für Mischungsverhältnisse in einem elektronischen Speicher hinterlegt oder können aufgrund der obigen Berechnungen in einen solchen Speicher geschrieben werden. Auf diese Weise steht dem Anwender eine Datenbank für die verschiedenen Mischungskombinationen zur Verfügung, die er bei der jeweiligen, gerade zu verstreckenden Partie abrufen kann.

Zur Eingabe der Mischungsverhältnisse weist die Spinnereivorbereitungsmaschine zweckmäßigerweise eine entsprechend ausgebildete Eingabeeinheit sowie eine Prozessoreinheit zur Bestimmung der Kalibrierungskurven aus den eingegebenen Mischungsverhältnissen auf.

Als Kalibrierungsmittel werden bevorzugt textile Faserbänder in Strangform mit definiertem Feuchtegehalt verwendet. Hierzu bieten sich beispielsweise konditionierte Proben an, bei denen die Faserfeuchte genau bekannt ist. Alternativ wird das gesamte Fasermaterial unter gleichen Raumbedingungen gelagert. Hierbei wird als Kalibrierungsmittel ein Teil dieses Fasermaterials verwendet. Es ist auch möglich, dass diese beiden Vorgehensweisen kombiniert werden.

Alternativ wird das zu verstreckende Material bei normalen Produktionsbedingungen in definierter Länge gewogen, anschließend getrocknet und in diesem Zustand wieder gewogen. Aus einem Vergleich dieser Bandfeinheiten wird der Feuchtegehalt bestimmt. Die Bandfeinheiten und der errechnete Feuchtegehalt werden anschließend der Auswerteeinheit des Mikrowellensensors zur Verfügung gestellt. Es können auch Bandmassen statt Bandfeinheiten verarbeitet werden. Die Kalibrierungskurve wird aus den gewogenen Bandfeinheiten und den dazugehörigen ermittelten Resonatorkennwerten, d. h. Frequenzverschiebung A und Feuchtigkeitsgehalt Φ, bestimmt. Die im Wesentlichen lineare, in der Regel durch den Nullpunkt verlaufende Funktion "Frequenzverschiebung aufgetragen gegen Bandfeinheit" wird hierbei bei normalen Produktionsbedingungen mit dem Mikrowellensensor gemessen und der aus der Wägung errechneten Faserfeuchte zugeordnet. Vorteilhafterweise wird mindestens ein zweiter Messpunkt von dem gleichen Material mit einem unterschiedlichen Feuchtegehalt ermittelt. Hiermit können unterschiedliche Feuchtegehalte während des Produktionlaufs ermittelt werden.

Bei einer weitergehenden Kalibrierung wird der Mikrowellen-Ausgangssensor aufgrund von Labormessungen nachkalibriert, bei denen beispielsweise die Ist-Bandfeinheit (und/oder die Bandfeuchtigkeit) des verstreckten Faserbands gemessen wird (Plausibilitätskontrolle). Auf Basis dieser Nachkalibrierung, d. h. der aktuellen Kennlinie des Ausgangssensors, wird der Mikrowellen-Eingangssensor vorteilhafterweise unter besonderer Berücksichtigung der unterschiedlichen Faserband-Temperaturen am Ein- und Auslauf und weiteren Einflüssen, z. B. Verschmutzungsgrad des Sensors, nachkalibriert. Dies kann beispielsweise dann von Vorteil sein, wenn das einlaufende und das auslaufende Faserbandmaterial unterschiedliche Temperaturen aufweisen, welche die Messergebnisse beeinflussen. Die Nachkalibrierung wird bevorzugt automatisch mit Hilfe eines Mikroprozessors vorgenommen.

Für die schnelle Kalibrierung des Mikrowellensensors werden vorzugsweise definierte textile Proben in Polymerverbindungen mit bekannten Massen eingegossen. Alternativ werden Ausgangspolymere der betreffenden Faserstoffe (Filamentgarne) verwendet, beispielsweise Viskoseschmelzen. Die Proben weisen vorzugsweise einen unterschiedlichen, bekannten Feuchtegehalt auf.

Vorteilhafterweise werden Proben zur Kalibrierung des mindestens einen Mikrowellensensors herangezogen, welche eine nahezu gleiche Dielektrizitätskonstante wie das zu verarbeitende Faserbandmaterial aufweisen.

Vorzugsweise wird eine einzige Auswerteelektronik für alle Resonatoren am Streckwerkseinlauf und/oder Streckwerksauslauf eingesetzt.

Es kommt einer präzisen Faserbanddickenmessung zugute, wenn das Faserbandmaterial vorteilhafterweise derart verdichtet wird, dass eine möglichst homogene Materialverteilung im Messschlitz des mindestens einen Mikrowellensensors vorliegt. Die Verdichtungsmittel können beispielsweise pneumatische Verdichtungsmittel sein. Vorzugsweise sind die Verdichtungsmittel jedoch zumindest teilweise als mechanische Führungselemente ausgebildet, beispielsweise in Form von Rundstäben, an deren Rundflächen das Faserbandmaterial entlang gleiten kann, oder in Form eines offenen oder geschlossenen Trichters. Zur Realisierung einer solchen Verdichtung sind zwei Varianten bevorzugt, wobei der Verdichtungsgrad je nach einlaufender Bandmasse vorzugsweise einstellbar bzw. verstellbar ist.

Bei der einen Variante sind die Verdichtungsmittel derart ausgebildet, dass die Verdichtungsmittel unmittelbar vor und/oder nach dem Sensor angeordnet sind. Der Sensor kann hierbei am Verdichtungsmittel bzw. umgekehrt angebracht sein.

Alternativ oder zusätzlich kann mindestens ein Führungselement, vorzugsweise aus elektrisch nicht-leitendem Werkstoff (z. B. Keramik), innerhalb des Sensors angebracht sein. Es sind vorzugsweise gerade, aber auch keilförmige oder bogenförmige Begrenzungen möglich.

Es ist vorteilhaft, dass eine gezielte Verdichtung des Faserbandmaterials zu Selbstreinigungseffekten des Messschlitzes bzw. des Resonatorraums führt.

Bei der Materialeinführung in den Messschlitz ist darauf zu achten, dass es zu keiner Überkreuzung der Faserbänder kommt. Hierzu können z. B. Rechen vor und/oder nach dem Sensor angeordnet sein.

In einer vorteilhaften Ausführungsform sind vor dem mindestens einen Sensor ein Trichter zum Verdichten bzw. Führen des oder der Faserbänder. Hinter dem mindestens einen Sensor können ein oder mehrere Rechen zum Verhindern der Faserband-Überkreuzung angeordnet sein.

Vorteilhafterweise ist unmittelbar hinter dem mindestens einen, vor dem Streckwerk angeordneten Mikrowellensensor ein Abzugswalzenpaar angeordnet, das sich über die Faserbandbreite erstreckt. Die Längsachsen der Walzen verlaufen demnach quer zur Faserbandlaufrichtung. Somit können das bzw. die Faserbänder, aus dem Sensor abgezogen werden, ohne wesentlich in der Breite komprimiert zu werden.

Besonders bevorzugt ist das Abzugswalzenpaar als Eingangswalzenpaar des dem Sensor nachgeordneten Streckwerks ausgebildet. Dieses Walzenpaar übernimmt somit die Doppelfunktion des Abzugs des mindestens einen Faserbands sowie der Teilnahme an der Verstreckung.

Bei Verschmutzungen des Mikrowellenresonators ist zwischen zwei Arten von Verschmutzungen zu unterscheiden. Zum einen sind dies leicht entfernbare Verschmutzungen, wie beispielsweise Faserflug, zum anderen schwer entfernbare Verschmutzungen, wie beispielsweise Honigtau und Avivage. Diese beiden Verschmutzungen führen zur Veränderung der Resonator-Kennwerte, so dass bevorzugt eine Reinigung des oder der Resonatoren vorgeschlagen wird.

Eine Schmutzentfernung kann in regelmäßigen Abständen stattfinden, beispielsweise und bevorzugt im Stoppzustand der Maschine. Die entsprechenden Reinigungseinrichtungen zur Reinigung des oder der Mikrowellenresonatoren von leicht oder schwer zu entfernenden Verschmutzungen können bei Überschreiten von vordefinierten Grenzwerten, z. B. hinsichtlich der Resonator-Kennwerte im leeren Zustand des Resonators, oder bei Überschreiten von Schmutz- oder Schmierfilmdicken, mittels geeigneter Steuermittel ausgelöst und/oder die Notwendigkeit einer Reinigung angezeigt werden. Die Reinigung kann entweder manuell oder mit Hilfe einer Reinigungseinrichtung durchgeführt werden, wobei die manuelle Reinigung bei den schwer zu entfernenden Verschmutzungen in einigen Fällen unabdingbar sein kann.

Die leichter entfernbaren Verschmutzungen lassen sich vorzugsweise durch Druckluft entfernen, wobei eine oder mehrere Luftdüsen auf den Messschlitz des Resonators gerichtet sind.

Vorzugsweise sind Steuerungsmittel vorhanden, welche bei schwer oder nicht zu entfernenden Verschmutzungen ein Stoppen der Maschine bedingen. Bevorzugt wird jedoch aus Gründen der Produktivität die Reinigung - sowohl der leichter als auch der schwerer zu entfernenden Verschmutzungen ― bei einem Kannenwechsel am Streckwerksauslauf oder einem Vorlagekannenwechsel vorgenommen, da dann die Maschine in der Regel kein Faserband produziert (außer bei einem sog. fliegenden Wechsel). Die Steuerungsmittel können in einem zentralen Maschinenrechner integriert sein.

Für die Reinigung ist der Mikrowellensensor bevorzugt ausfahrbar ausgebildet, beispielsweise mittels eines Motors und einer Laufschiene, auf der der Sensor verfahrbar ist, wobei das Faserbandmaterial bevorzugt in seiner Lage unverändert bleibt und vorzugsweise in dieser Position mittels geeigneter Haltemittel fixiert ist. Die Reinigung des Sensors erfolgt bevorzugt mittels Druckluft oder mechanischer Reinigungsmittel, welche die Resonatorauskleidung ― z. B. Keramik ― schonen. Bei einem stationär ausgebildeten Sensor sind die Verschmutzungen manuell oder automatisch (mittels Druckluft, mechanischer Reinigungsmittel etc.) aus dem Messschlitz zu entfernen. Nach der Reinigung mit beispielsweise Druckluft kann vorzugsweise mittels einer elektronischen Auswerteeinheit durch Auswertung der Resonator-Kennwerte im leeren Zustand (Güte) festgestellt werden, ob Schmutz noch anhaftet oder nicht, wobei die Grenzwerte für schwer entfernbares Material zu beachten sind.

Die Steuerungsmittel zur Steuerung der Sensorenreinigung können in einem zentralen Maschinenrechner integriert sein.

Um den Verschmutzungsgrad des Resonators zu minimieren, wird der Messraum vorzugsweise derart konstruktiv gestaltet, dass eine Anhaftung von Verschmutzungen vermindert oder sogar verhindert wird. Es bietet sich hierbei vorzugsweise an, die Innenoberfläche des Sensors aus schmutzabweisenden und abriebfesten Werkstoffen zu gestalten und/oder scharfe Kanten zu vermeiden, insbesondere an den Ein- und Auslaufstellen des Faserbandmaterials in den Sensor.

Die Positionierung eines Mikrowellensensors am Einlauf der Spinnereivorbereitungsmaschine ist auf verschiedene Weise realisierbar. Einerseits ist eine unmittelbare Anordnung vor dem Streckwerk möglich. In diesem Fall kann das Eingangswalzenpaar hinter dem Sensor derart angeordnet und ausgebildet sein, dass das Eingangswalzenpaar des Streckwerks zur Förderung des Materials durch den Messschlitz des Sensors eingesetzt wird (s. o.). Vorteilhaft ist dann der Abstand zwischen Sensor und Eingangswalzenpaar kleiner als die mittlere Stapellänge, um unkontrollierte Faserbewegungen bei diesem Förderprozess zu vermeiden.

In alternativen oder zusätzlichen Ausbildungen kann ein Mikrowellensensor im Vorverzugsfeld des Streckwerks, gebildet von Eingangs- und Mittelwalzenpaar, und/oder im Hauptverzugsfeld des Streckwerks, gebildet von Mittel-und Lieferwalzenpaar, angeordnet werden.

Zur Positionierung eines Mikrowellensensors am Auslauf der Spinnereivorbereitungsmaschine sind ebenfalls mehrere Möglichkeiten vorhanden. So ist beispielsweise eine Anordnung zwischen einer dem Streckwerk nachgeschalteten Vliesdüse und einem weiter stromabwärts angeordneten Kalanderwalzenpaar möglich.

Auch ist eine Ausbildung des Sensors als Vliesdüseneinsatz vorteilhaft, wobei der Sensor hierbei die Funktion eines Bandformers übernehmen würde. Es bietet sich bei einer solchen Ausgestaltung an, den Sensor in geschlossener Form ― z. B. im Querschnitt zylinderförmig - auszubilden. Es sind selbstverständlich auch andere geometrische Formen möglich, z. B. eine im Querschnitt elliptische oder rechteckige Ausbildung. In den Vliesdüseneinsatz lässt sich auch vorzugsweise eine Einfädelfunktion für das verstreckte Faserband integrieren, welche beispielsweise mittels Luftdüsen realisiert ist. Alternativ kann die Vliesdüse in den Mikrowellensensor integriert sein.

Eine weitere bevorzugte Ausbildung sieht eine Positionierung eines Mikrowellensensors unmittelbar nach dem Ausgangswalzenpaar des Streckwerks vor. In diesem Fall kann der Sensor offen ausgebildet sein, beispielsweise in Form eines gabelförmigen Schlitzes. Eine Bandumformung erfolgt dann durch eine nachgeschaltete Vliesdüse.

Ein Mikrowellensensor kann ebenfalls zwischen Kalanderwalzenpaar und Drehteller positioniert werden.

Bevorzugt weist die Spinnereivorbereitungsmaschine Einfädelmittel zur automatischen Einfädelung des Faserbandmaterials in den mindestens einen Sensor bei der Verarbeitung von neuen Partien bzw. Behebung von Bandbrüchen auf. Derartige Einfädelmittel umfassen beispielsweise eine oder mehrere Luftdüsen, so dass das Faserbandmaterial von dem erzeugten Luftstrom erfasst und in den Sensor eingeführt wird. Alternativ oder zusätzlich können die Einfädelmittel auch auf mechanischer Basis arbeiten, beispielsweise durch Klemmung und Bewegung bzw. Einführung des oder der Faserbänder in den Messschlitz des Resonators.

Die Einfädelmittel können weiterhin mechanisch wirkende Haltemittel - beispielsweise Klemmen ― umfassen, mit denen bei Reinigungsarbeiten (s. o.) das Faserbandmaterial nach Ausfahren des Sensors aus einer Messposition in eine Reinigungsposition in einer definierten Position gehalten werden kann. Auf diese Weise kann anschließend das Material ohne manuellen Eingriff in den Messschlitz des wieder in Messposition verfahrenen Sensors eingeführt werden.

Des Weiteren wurde erkannt, dass das zu verarbeitende Fasermaterial am Einlauf und Auslauf der Strecke unterschiedliche Temperaturverläufe aufweisen kann, welche die Messergebnisse verfälschen können. Diesbezüglich wird vorgeschlagen, dass die Spinnereivorbereitungsmaschine eine Einrichtung zur Temperaturmessung aufweist, um die Temperaturen des textilen Fasermaterials mit mindestens jeweils einem Temperaturfühler bevorzugt fortlaufend (inklusive Start/Stoppphase und insbesondere Kaltstart) zu ermitteln und damit die Messergebnisse zu kompensieren. Dies kann entsprechend einer vorteilhaften Ausführungsform in der Messelektronik des Mikrowellensensors geschehen oder durch externe Kompensation. Auf diese Weise können insbesondere Kreuzkorrelationen der Messergebnisse am Einlauf und Auslauf vorgenommen werden, wobei vorzugsweise die unterschiedlichen Bandgeschwindigkeiten am Ein- und Auslauf sowie die Laufzeit zwischen den beiden Sensoren berücksichtigt werden, z. B. in einem zentralen Rechner der Maschine. Je nach Temperaturdifferenz zwischen Material am Einlauf und Auslauf soll eine Korrektur vorgenommen werden (Offset-Korrektur).

Bei der Verwendung von Sensoren auf Mikrowellenbasis ist die elektrische Leitfähigkeit von Mattierungselementen und Pigmenten in zu verstreckender oder verstreckter Baumwolle in den meisten Fällen unbedenklich. Bei elektrisch leitenden Werkstoffen, wie z. B. Kohlefasern, kann ggf. derselbe Mikrowellensensor eingesetzt werden. Gleichfalls kann ein zweiter Sensor verwendet werden, der vorzugsweise nach einem anderen physikalischen Prinzip arbeitet.

Werden jeweils mindestens zwei Resonatoren an einer Messposition ― d. h. entweder am Einlauf oder am Auslauf ― hintereinander geschaltet, kann mit ihnen bevorzugt ein Bandfilter realisiert werden.

Gemäß einem weiteren Aspekt ist mindestens ein Mikrowellensensor zur Messung der Faserbanddicke am Einlauf und mindestens ein Mikrowellensensor zur Messung der Faserbanddicke am Auslauf vorgesehen, wobei die Sollbandfeinheit des die Maschine verlassenden Faserbands vorgebbar ist, beispielsweise über ein Maschinendisplay. Die Maschine ist derart ausgebildet, dass die vom mindestens einen Sensor am Einlauf und dem mindestens einen Sensor am Auslauf gemessenen Ist-Bandfeinheiten mit Hilfe einer Auswerteeinheit, die beispielsweise in einem zentralen Maschinenrechner integriert ist, miteinander korrelierbar und die Ergebnisse an eine Steuereinheit weitergebbar sind, um die Verzugsorgane gemäß der vorgegebenen Sollbandfeinheit entsprechend anzusteuern. Vorzugsweise wird mittels der Auswerteeinheit eine Kreuzkorrelation zwischen den von dem mindestens einen Sensor am Einlauf und dem mindestens einen Sensor am Auslauf gemessenen Ist-Bandfeinheiten vorgenommen. Eine anschließende Plausibilitätskontrolle ist vorteilhaft.

Die Erfindung lässt sich bei Karden, Strecken sowie Kämmmaschinen mit reguliertem als auch unreguliertem Streckwerk einsetzen. Auch ist ein Einsatz der Erfindung in einer Kombination aus einer Karde und nachgeschaltetem Streckwerk vorteilhaft.

Vorteilhafte Weiterbildungen der Erfindung sind durch die Merkmale der Unteransprüche gekennzeichnet.

Im Folgenden wird die Erfindung in ihren verschiedenen Aspekten anhand der Figuren näher erläutert. Es zeigen:
- **Figur 1**: eine Regulierstrecke mit Regulierungskomponenten in schematischer Darstellung;
- **Figur 2**: eine perspektivische Ansicht eines offenen Mikrowellensensors;
- **Figur 3**: eine Aufsicht auf den Einlauf einer Strecke mit einem geschlossenen Mikrowellensensor;
- **Figur 4**: eine Seitenansicht des Streckeneinlaufs der Figur 3;
- **Figur 5**: eine Aufsicht auf den Einlauf einer Strecke mit einem geschlossenen Mikrowellensensor gemäß einer zweiten Ausführungsform, und
- **Figur 6**: eine Seitenansicht der Ausführungsform der Figur 5.

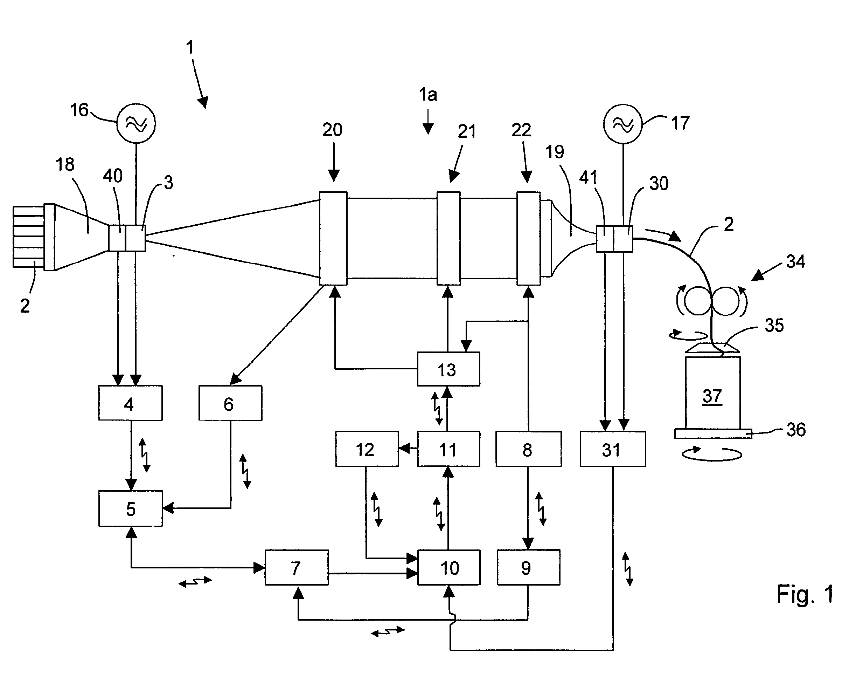

In Figur 1 ist schematisch ein beispielhaftes Regulierungsprinzip an einer Strecke 1 dargestellt. Am Eingang der Strecke 1 wird die Banddicke von einlaufenden Faserbändern 2 ― in diesem Fall sechs Faserbänder 2 ― mit einem Mikrowellensensor 3, der nach dem Resonatorprinzip arbeitet, erfasst. Der Sensor 3 ist mit einem Mikrowellengenerator 16 verbunden, der von einer (nicht dargestellten) Prozessoreinheit angesteuert wird und Mikrowellen in den Resonator des Sensors 3 einkoppelt. Dem Sensor 3 ist ein als Verdichtungsmittel ausgebildeter Trichter 18 zum Verdichten der Faserbänder 2 vorgeschaltet. Nach Passieren des Mikrowellensensors 3 werden die Faserbänder 2 wieder ausgebreitet, um in das Streckwerk 1a einzulaufen. Die Messwerte des Sensors 3 werden von einer Auswerteeinheit 4 in die Banddickenschwankungen repräsentierende elektrische Spannungswerte umgewandelt, die einem Speicher 5 zugeführt werden (elektrische Signale sind in der Figur 1 mit einem gezackten Doppelpfeil gekennzeichnet, während mechanische Signale keine spezielle Kennzeichnung aufweisen). Der Speicher 5 gibt die Messβspannung mit Unterstützung eines Impulsgebers bzw. Taktgebers 6 mit definierter zeitlicher Verzögerung an eine Sollwertstufe 7 weiter. Der Impulsgeber 6 erhält von einem Eingangswalzenpaar 20, das gleichzeitig zur Förderung der Faserbänder 2 durch den Sensor 3 dient, entsprechend der Bandlängenförderung durch dieses Walzenpaar 20 ein Trigger-Signal (sog. "vordefinierte konstante Abtastlänge"). Alternativ kann der Impulsgeber mit einem anderen Walzenpaar gekoppelt sein, beispielsweise mit einem (nicht dargestellten) Transportwalzenpaar unmittelbar hinter dem Sensor 3 (in Bandlaufrichtung gesehen). In einem solchen Fall dient nicht das Eingangswalzenpaar 20 zum Transport der Faserbänder 2 durch den Sensor 3, sondern das Transportwalzenpaar.

Die Sollwertstufe 7 erhält außerdem von einem Leittacho 9 eine Leitspannung, die ein Maß für die Drehzahl der unteren, von einem Hauptmotor 8 angetriebenen Walze eines Lieferwalzenpaares 22 ist. Anschließend wird in der Sollwertstufe 7 eine Sollspannung errechnet und an eine Steuereinheit 10 weitergegeben. In der Steuereinheit 10 findet ein Soll-lstwert-Vergleich statt, der dazu benutzt wird, einem Regelmotor 11 eine ganz bestimmte, der gewünschten Verzugsänderung entsprechende Drehzahl zu erteilen. Die Istwerte des Regelmotors 11 werden hierbei einem lstwert-Tacho 12 übermittelt, der die entsprechende Ist-Spannung dann an die Steuereinheit 10 weitergibt. Der Regelmotor 11 treibt in ein vom Hauptmotor 8 angetriebenes Planetengetriebe 13, das die Drehzahlen der unteren Walze des Eingangswalzenpaares 20 und der unteren Walze 21 eines Mittelwalzenpaares 21 derart verändert, dass eine Bandvergleichmäßigung bzw. -verstreckung stattfindet.

Als Regelgröße dient die Banddicke. Aufgrund des Faserbandtransports vom Sensor 3 zum Verzugsfeld - bestehend aus dem Eingangs-, Mittel- und Lieferwalzenpaar 20, 21, 22 (die Walzenpaare sind in der Aufsicht dargestellt) - wird eine Totzeit errechnet. Die Drehzahl für den Regelmotor 11 als Stellgrö-βe wird von der Steuereinheit 10 ermittelt, wobei hierbei die Ist-Dicke der Faserbänder 2, der Banddickensollwert (als Führungsgröße) und die Drehzahlen des Hauptmotors 8 und des Regelmotors 11, verarbeitet werden. Durch die proportionale Überlagerung der Drehzahlen des Hauptmotors 8 und des Regelmotors 11 und unter Berücksichtigung der genannten Totzeit wird die Banddicke im Streckwerk 1a im sog. Regeleinsatzpunkt geregelt.

Am Auslauf des Streckwerks 1 a ist ein mit einem weiteren Mikrowellengenerator 17 verbundener Resonator eines Mikrowellensensors 30 angeordnet, der in dem gezeigten Ausführungsbeispiel einem als Verdichtungsmittel ausgebildeten Bandtrichter bzw. einer Vliesdüse 19 nachgeschaltet ist. Die Signale des Sensors 30 werden einer Auswerteeinheit 31 zugeführt, die elektrische Spannungssignale entsprechende der Banddicke des verstreckten Faserbands 2 liefert und an die Steuereinheit 10 weitergibt. Alternativ oder zusätzlich dienen die Ergebnisse vom Sensor 30 lediglich zur Bandüberwachung und zur Bandqualitätskontrolle. Entsprechend werden diese Ergebnisse bevorzugt auf einem Display an der Maschine und/oder an einer zentralen Einheit in der Spinnerei angezeigt.

Andere Schaltungsanordnungen sind möglich, insbesondere der Einsatz eines zentralen Rechners.

Das verstreckte Faserband 2 wird mittels Kalanderwalzen 34 aus dem Mikrowellensensor 30 abgezogen (die Distanzen zwischen Sensor 30 und Walzen 34 sind nicht maßstäblich) und mit Hilfe eines in einem rotierenden Drehteller 35 angeordneten Bandkanals in eine Rundkanne 37 abgelegt, welche auf einem rotierenden Kannenteller 36 steht. Alternativ ist die Spinnkanne 36 als Flachkanne ausgebildet, die hin und her changiert.

Am Streckwerkseinlauf und am Streckwerksauslauf ist jeweils eine Einrichtung 40 bzw. 41 zur vorzugsweise kontinuierlichen Messung der Faserbandtemperatur angeordnet (in der Figur 1 in unmittelbarer Nähe zu dem jeweiligen Sensor 3 bzw. 30). Die Temperaturmesswerte bzw. die für die Temperatur charakteristischen Spannungswerte werden der jeweiligen Auswerteeinheit 4 bzw. 31 zugeführt, die hierbei zusätzlich die Funktion einer Kompensationseinheit zur Temperatur-Kompensation der Messergebnisse des jeweiligen Sensors 3 bzw. 30 übernimmt. Die Ergebnisse der Auswerteeinheiten 4 bzw. 31 sind beispielsweise mittels einer Kreuzkorrelation miteinander korrelierbar. Eine solche Korrelation kann beispielsweise in der Steuereinheit 10 vorgenommen werden, die hierzu entsprechende Rechenleistungen durchführen können muss. Alternativ sind separate Prozessoreinheiten oder eine gemeinsame Prozessoreinheit zur Temperaturkompensation und ggf. auch zur Korrelation der von der Einrichtung 40 und 41 stammenden Signale vorhanden.

Vorzugsweise beide Sensoren 3, 30 lassen sich automatisch reinigen, beispielsweise durch Druckluft aus einer oder mehreren Luftdüsen, die auf den Messschlitz des jeweiligen Sensors 3, 30 gerichtet sind. Entsprechende Steuereinrichtungen (nicht dargestellt) lösen eine derartige Reinigung aus, vorzugsweise in zeitlichen Abständen und/oder bei Überschreiten eines Grenzwertes der Resonator-Kennwerte (Resonatorgüte) im leeren Zustand und/oder bei Überschreiten von vorgegebenen Schmutz- oder Schmierfilmdicken. Derartige Luftdüsen können auch als Einfädelmittel zum automatischen Einfädeln des oder der zu messenden Faserbänder in den mindestens einen Sensor 3 bzw. 30 dienen.

In einer alternativen, nicht dargestellten Ausführungsform der Erfindung kann die Spinnereivorbereitungsmaschine Einzelantriebe aufweisen, die bevorzugt jeweils eigene Regelkreise besitzen und wobei vorteilhafterweise ein zentraler Rechner eingesetzt wird.

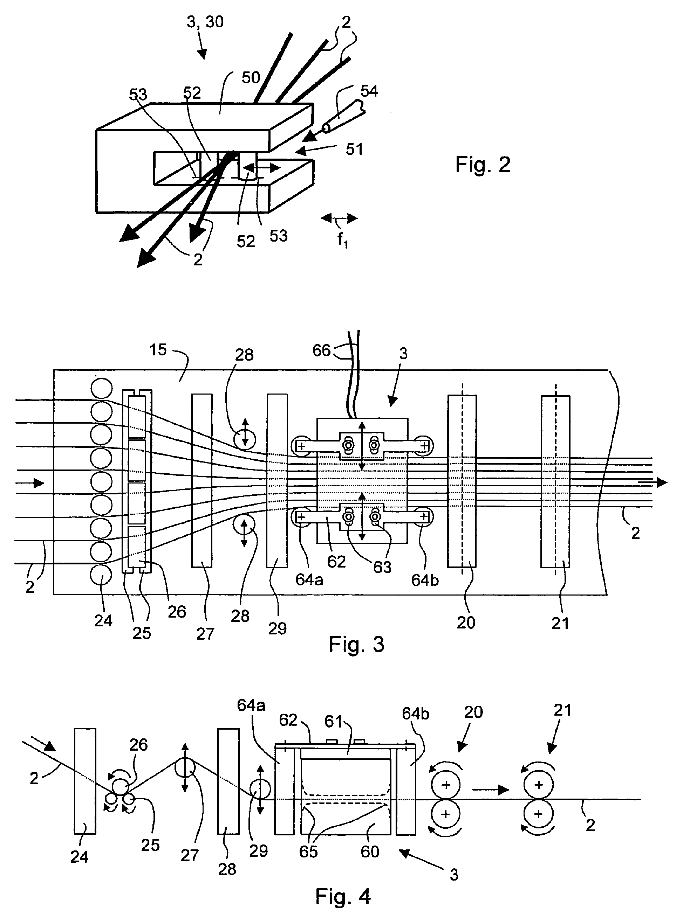

In Figur 2 ist ein offener Mikrowellensensor 3, 30 dargestellt, der aus einem U-förmig gebogenen Resonator 50 besteht, wobei die entsprechende Aussparung als Messschlitz 51 ausgebildet ist, durch den ein oder mehrere, schematisch als Pfeile angedeutete Faserbänder 2 führbar sind. In dem Messschlitz 51 ist zu beiden Seiten des oder der Faserbänder 2 je ein Rundstab 52 angeordnet, wobei beide Rundstäbe 52 zusammen als verdichtende Führungselemente dienen. Die Rundstäbe 52 sind auf schematisch angedeuteten Führungen 53 in Bandquerrichtung verschieblich (s. Doppelpfeil) und in der jeweiligen Position vorzugsweise fixierbar angeordnet. Um das oder die Faserbänder 2 in den Messschlitz 51 und zwischen die beiden Rundstäbe 52 einzuführen, sind ein oder mehrere Luftdüsen 54 vorgesehen, die im Wesentlichen in Bandlaufrichtung ausgerichtet sind (in der Figur 2 läuft diese auf den Betrachter zu) und durch dementsprechende Druckluft Faserband in Bandlaufrichtung mitnehmen (s. Pfeil). Des Weiteren ist durch den Doppelpfeil f1 angedeutet, dass der gesamte Sensor 3, 30 von einer Messposition in eine Reinigungsposition und wieder in die Messposition verfahrbar ausgebildet sein kann.

In den Figuren 3 - 6 sind zwei weitere Ausführungsformen eines am Einlauf eines Streckwerks angeordneten Mikrowellensensors 3 dargestellt. In der Aufsicht gemäß der Figur 3 ist ein Einlauftisch 15 zu sehen, an dessen den (nicht dargestellten) Vorlagekannen zugewandten Ende eine Rechenanordnung 24 vorgesehen ist. Die Rechenanordnung 24 besteht aus neun vertikal aufgestellten Rundstäben, zwischen denen acht Faserbänder 2 aus den Vorlagekannen zum Mikrowellensensor 3 geführt werden (die Faserbänder 2 sind in der Seitenansicht der Figur 4 gepunktet dargestellt, wenn sie verdeckt verlaufen). Der Rechenanordnung 24 nachgeordnet sind zwei parallel verlaufende angetriebene Transportwalzen 25, auf die vier miteinander fluchtende Jockey- bzw. Belastungswalzen 26 gelegt sind. Zwischen jeder der Jockeywalzen 26 und den darunter angeordneten Transportwalzen 25 sind jeweils zwei der acht Bänder 2 geführt. Bei einem Bandbruch wird ein elektrischer Kontakt zwischen der betreffenden Jockeywalze 26 und den Transportwalzen 25 geschlossen und für einen Bediener erkennbar angezeigt.

In Bandtransportrichtung folgt ein horizontal verlaufender Rundstab 27, der unbeweglich oder drehbar ausgebildet ist und über den die Faserbänder 2 geführt sind. Zudem ist durch den vertikalen Doppelpfeil in der Figur 4 angedeutet, dass sich der Rundstab 27 in der Höhe vorzugsweise verstellen lässt. Dem Rundstab 27 nachgeordnet sind zwei aufrecht aufgestellte, im Querschnitt kreisförmig ausgebildete Führungselemente 28, zwischen denen die Faserbänder 2 hindurchlaufen. Der Abstand zwischen den beiden Führungselementen 28 ist bevorzugt einstellbar, wie dies durch die entsprechenden Doppelpfeile in der Figur 3 angedeutet ist.

An die Führungselemente 28 schließt sich in Bandlaufrichtung ein zweiter horizontal verlaufender Rundstab 29 an, der wiederum unbeweglich oder drehbar gelagert sein kann und unter den die Faserbänder 2 geführt werden. Der Rundstab 29, der gemäß Figur 4 höheneinstellbar ausgebildet ist (s. Doppelpfeil), dient zur mittigen Einführung der Faserbänder 2 in den Resonator 60 des Mikrowellensensors 3 (s. Figur 4). Zwischen dem Rundstab 29 und dem Sensor 3 sind noch zwei weitere aufrechte und zu beiden Seiten der Faserbänder 2 angeordnete Führungselemente 64a mit rundem Querschnitt vorgesehen. Diese sind oberseitig an horizontal in Bandlaufrichtung verlaufenden Flachstäben 62 befestigt, die ihrerseits mittels in zwei jeweils Langlöchern 63 gelagerten Schrauben an einer Deckplatte 61 befestigt sind. Die Deckplatte 61 ist auf dem Resonator 60 angebracht. An der Bandaustrittsseite des Sensors 3 sind auf gleiche Weise befestigte Führungselemente 64b vorgesehen. Der Abstand der Führungselemente 64a, 64b lässt sich durch Verschieben der Flachstäbe 62 in den Langlöchern 63 einstellen.

Am Eingang und am Ausgang des Sensors 3, dessen Anschlusskabel 66 (an einen Mikrowellengenerator und an einen Signalempfänger) in Figur 3 ebenfalls dargestellt sind, sind jeweils horizontal verlaufende, sich entlang der Faserbandbreite erstreckende, abgerundete Kanten 65 als weitere Führungselemente angeordnet (s. Figur 4, in gestrichelten Linien dargestellt). Diese Führungselemente 65 befinden sich demnach im Sensor 3 und dienen zur gleichmäßigen Führung der Faserbänder 2 durch den Resonator 60.

Den Führungselementen 64b nachgeordnet ist ein Transportwalzenpaar 20, welches die Faserbänder 2 aus dem Sensor 3 abzieht. Vorteilhafterweise ist dieses Walzenpaar als Eingangswalzenpaar 20 des nachfolgenden Streckwerks 1a ausgebildet (vgl. Figur 1). Im Gegensatz zu mechanisch abtastenden, die Faserbänder 2 komprimierenden Banddickenmesseinrichtungen, bei denen die Faserbänder 2 vor Eintritt in das Streckwerk 1 a wieder ausgebreitet werden müssen, kann vorliegend auf eine solche raumgreifende Ausbreitung verzichtet werden. Die gesamte Maschine lässt sich somit wesentlich kompakter gestalten. Das Einzugswalzenpaar 20 dient nach alledem einerseits zum Abzug der Bänder 2 aus dem Sensor 3 und andererseits als Verstreckungsorgan. Die Faserbänder 2 laufen im Wesentlichen parallel zueinander durch den Sensor und weiter ins Streckwerk 1 a ein.

Die Führungselemente 28, 64a, 64b zur seitlichen Führung der Faserbänder 2 können alternativ oder zusätzlich zu ihrer linearen Verstellbarkeit in Bandquerrichtung (s. Figur 3) einen exzentrischen Querschnitt unter Vermeidung scharfer Umfangskanten aufweisen (nicht dargestellt). Um die Durchtrittsbreite für die Faserbänder 2 zu verändern, lassen sich diese Führungselemente 28, 64a, 64b um ihre Längsachse drehen und in ihrer jeweiligen Position arretieren.

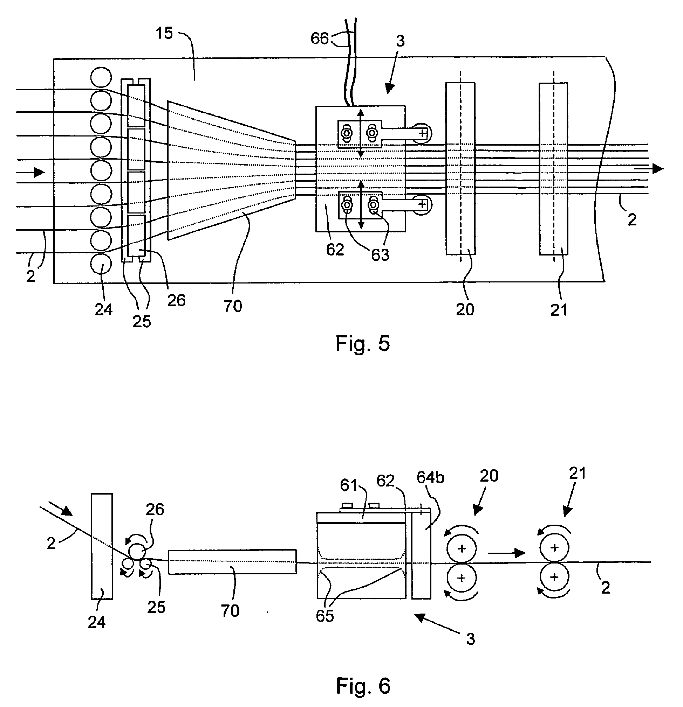

Gemäß der in den Figuren 5 und 6 dargestellten Ausführungsform ist vor dem Sensor 3 ein Trichter 70 angeordnet, durch den die Faserbänder 2 im Anschluss an die Jockey- und Transportwalzen 26, 25 geführt werden. Der Trichter 70 kann vorteilhafterweise in seiner Breite eingestellt werden, bevorzugt in seiner Einlauf- und/oder in seiner Auslaufbreite. Die Ausführungsform gemäß der Figuren 5 und 6 entspricht im Übrigen derjenigen der Figuren 3 und 4. Zu erwähnen sind die Führungselemente 64b am Ausgang des Sensors 3, da die Führungselemente 64a am Sensoreingang wegen des Trichters 70 hinfällig sind. Wenn die Ausgangsbreite des Trichters 70 hingegen nicht einstellbar ist, können Führungselemente 64a jedoch vorteilhafterweise vorhanden sein.

## Patentansprüche

1. Spinnereivorbereitungsmaschine mit einem regulierten Streckwerk (1 a), welchem mindestens ein Faserband (2) zur Verstreckung vorgelegt wird, mit mindestens einem Mikrowellensensor (3, 30) mit einem Resonator (50) zur Faserbanddickenmessung am Streckwerkseinlauf und/oder am Streckwerksauslauf, **dadurch gekennzeichnet, dass** der mindestens eine Sensor (3, 30) anhand von Kalibrierungskurven für unterschiedliche Faserbandmaterialien und/oder Faserbandmischungen kalibrierbar ist.

2. Spinnereivorbereitungsmaschine nach Anspruch 1, **dadurch gekennzeichnet, dass** die Kalibrierungskurven in der Messelektronik speicherbar und/oder bei Bedarf von externen Medien abrufbar sind.

3. Spinnereivorbereitungsmaschine nach Anspruch 1 oder 2, **dadurch gekennzeichnet, dass** für unterschiedliche Faserbandmaterialien mindestens je eine Kalibrierungskurve erstellbar und abrufbar ist, wobei zwischen den verschiedenen Kalibrierungskurven umgeschaltet werden kann.

4. Spinnereivorbereitungsmaschine nach einem der Ansprüche 1 bis 3, **dadurch gekennzeichnet, dass** bei Faserbandmischungen aus vorgegebenen Mischungsverhältnissen eine neue Kalibrierungskurve bestimmbar ist.

5. Spinnereivorbereitungsmaschine nach Anspruch 4, **dadurch gekennzeichnet, dass** die neue Kalibrierungskurve über Mittelung, Interpolation und/oder Regression von mindestens zwei Kalibrierungskurven bestimmbar ist.

6. Spinnereivorbereitungsmaschine nach einem der Ansprüche 1 bis 5, **dadurch gekennzeichnet, dass** für die Bestimmung der neuen Kalibrierungskurve Daten, vorzugsweise in Form von Tabellen, bezüglich der Kalibrierungskurven von unterschiedlichen Faserbandmaterialien in einem elektronischen Speicher hinterlegbar sind.

7. Spinnereivorbereitungsmaschine nach einem der Ansprüche 1 bis 6, **gekennzeichnet durch** eine Eingabeeinheit zur Eingabe der Mischungsverhältnisse sowie einer Prozessoreinheit zur Bestimmung einer Kalibrierungskurve aus den eingegebenen Mischungsverhältnissen.

8. Spinnereivorbereitungsmaschine nach einem der Ansprüche 1 bis 7, **dadurch gekennzeichnet, dass** ein Mikrowellensensor (3) am Streckwerkseinlauf durch einen nachkalibrierten Mikrowellensensor (30) am Streckwerksauslauf mit Hilfe eines Mikroprozessors nachkalibrierbar ist.

9. Verfahren zur Kalibrierung einer Spinnereivorbereitungsmaschine nach Anspruch 8, **dadurch gekennzeichnet, dass** der Mikrowellen-Ausgangssensor (30) aufgrund von Labormessungen nachkalibriert wird, bei denen beispielsweise die Ist-Bandfeinheit des verstreckenden Faserbands gemessen wird, und dass auf Basis dieser Nachkalibrierung der Mikrowellen-Eingangssensor (3) automatisch oder manuell nachkalibriert wird.

10. Verfahren zur Kalibrierung einer Spinnereivorbereitungsmaschine nach einem der Ansprüche 1 bis 8, **dadurch gekennzeichnet, dass** als Kalibrierungsmittel strangförmige textile Faserbänder mit definiertem Feuchtegehalt verwendet werden oder dass das gesamte Faserbandmaterial unter gleichen Raumbedingungen gelagert wird und ein Teil dieses Faserbandmaterials zur Kalibrierung herangezogen wird.

11. Verfahren nach Anspruch 10, **dadurch gekennzeichnet, dass** die textilen Kalibrierungsproben in aushärtende Materialien, beispielsweise Polymere, eingegossen oder Ausgangspolymere der entsprechenden Faserstoffe, beispielsweise Viskoseschmelze, verwendet werden.

12. Verfahren nach Anspruch 10 oder 11, **dadurch gekennzeichnet, dass** textile Kalibrierungsproben verwendet werden, die möglichst die gleiche Dielektrizitätskonstante wie das zu verarbeitende Faserbandmaterial aufweisen.

## Claims

1. Spinning preparation machine with autoleveling drafting system (1 a), to which at least one fibre sliver (2) is presented for drafting, with at least one microwave sensor (3, 30) with a resonator (50) to measure fibre sliver thickness at the drafting system input and/or at the drafting system output, **characterized in that** the at least one sensor (3, 30) can be calibrated on basis of calibration curves for different fibre sliver materials and/or fibre sliver mixtures.

2. Spinning preparation machine as in claim 1, **characterized in that** the calibration curves can be stored in the electronic measuring system and/or can be called up by external media when needed.

3. Spinning preparation machine as in claim 1 or 2, **characterized in that** for different fibre sliver materials at least one calibration curve can be provided and called up for each fibre sliver material, it being possible to switch over among the different calibration curves.

4. Spinning preparation machine as in one of the claims 1 to 3, **characterized in that** a new calibration curve can be determined on basis of predetermined mixture ratios in case of fibre sliver mixtures.

5. Spinning preparation machine as in claim 4, **characterized in that** the new calibration curve can be determined by taking the mean, interpolation and/or regression of at least two calibration curves.

6. Spinning preparation machine as in one of the claims 1 to 5, **characterized in that** data, preferably in form of tables pertaining to the calibration curves of different fibre sliver materials, can be stored in an electronic memory to determine new calibration curves.

7. Spinning preparation machine as in one of the claims 1 to 6, **characterized by** an input unit for the input of the mixture ratios as well as by a processor unit to determine a calibration curve from the entered mixture ratios.

8. Spinning preparation machine as in one of the claims 1 to 7, **characterized in that** a microwave sensor (3) at the drafting system input can be post-calibrated by a post-calibrated microwave sensor (30) at the drafting system output by means of a microprocessor.

9. Process for the calibration of a spinning preparation machine as in claim 8, **characterized in that** the microwave output sensor (30) is post-calibrated based on laboratory measurements in which e.g. the actual sliver fineness of the drafted fibre sliver is measured, and **in that** the microwave input sensor (3) is post-calibrated automatically or manually based on this post-calibration.

10. Process for the calibration of a spinning preparation machine as in one of the claims 1 to 8, **characterized in that** skein shaped textile fibre slivers with defined moisture content are used for calibration, or **in that** the entire fibre sliver material is stored under uniform environmental conditions and part of this fibre sliver material is used for calibration.

11. Process as in claim 10, **characterized in that** the textile calibration samples are poured into hardening materials, e.g. polymers or **in that** base polymers of the corresponding fibre materials, e.g. melted viscose are used.

12. Process as in claim 10 or 11, **characterized in that** textile calibration samples possessing as much as possible the same relative permittivity as the fibre sliver material to be processed are used.

## Revendications

1. Machine de préparation de filage avec un banc d'étirage contrôlé (1a), auquel est alimenté au moins un ruban de fibres (2) en vue de l'étirage, avec au moins un capteur à microondes (3, 30) avec un résonateur (50) pour la mesure de l'épaisseur du ruban de fibres à l'entrée du banc d'étirage et/ou à la sortie du banc d'étirage, **caractérisée en ce que** l'au moins un capteur (3, 30) peut être calibré pour des matières et/ou des mélanges de ruban de fibres différents à l'aide de courbes d'étalonnage.

2. Machine de préparation de filage selon la revendication 1, **caractérisée en ce que** les courbes d'étalonnage peuvent être mémorisées dans l'électronique de mesure et/ou appelées en cas de besoin depuis des supports externes.

3. Machine de préparation de filage selon la revendication 1 ou 2, **caractérisée en ce qu'**au moins une courbe d'étalonnage respective peut être créée et appelée pour des matières de ruban de fibres différentes, sachant qu'il est possible d'effectuer une commutation entre les différentes courbes d'étalonnage.

4. Machine de préparation de filage selon l'une des revendications 1 à 3, **caractérisée en ce qu'**une nouvelle courbe d'étalonnage peut être déterminée pour des mélanges de rubans de fibres présentant des rapports de mélange prédéfinis.

5. Machine de préparation de filage selon la revendication 4, **caractérisée en ce que** la nouvelle courbe d'étalonnage peut être déterminée par formation d'une moyenne, d'une interpolation et/ou d'une régression d'au moins deux courbes d'étalonnage.

6. Machine de préparation de filage selon l'une des revendications 1 à 5, **caractérisée en ce que** pour la détermination de la nouvelle courbe d'étalonnage, des données sur les courbes d'étalonnage de matières de rubans de fibres différentes, de préférence sous forme de tableaux, peuvent être stockées dans une mémoire électronique.

7. Machine de préparation de filage selon l'une des revendications 1 à 6, **caractérisée par** une unité de saisie pour la saisie des rapports de mélange ainsi qu'une unité de processeur pour la détermination d'une courbe d'étalonnage sur la base des rapports de mélange saisis.

8. Machine de préparation de filage selon l'une des revendications 1 à 7, **caractérisée en ce qu'**un capteur à microondes (3) à l'entrée du banc d'étirage peut être réétalonné à l'aide d'un microprocesseur par un capteur à microondes réétalonné (30) à la sortie du banc d'étirage.

9. Procédé pour étalonner une machine de préparation de filage selon la revendication 8, **caractérisé en ce que** le capteur à microondes de sortie (30) est réétalonné sur la base de mesures en laboratoire, qui consistent par exemple à mesurer la finesse réelle du ruban de fibres à étirer, et que le capteur à microondes d'entrée (3) est réétalonné automatiquement ou manuellement sur la base de ce réétalonnage.

10. Procédé pour étalonner une machine de préparation de filage selon l'une des revendications 1 à 8, **caractérisé en ce que** des rubans de fibres textiles en forme de cordes avec une teneur en humidité définie sont utilisés en tant que moyens d'étalonnage ou que l'ensemble de la matière du ruban de fibres est stockée dans des conditions ambiantes identiques et une partie de cette matière de rubans de fibres est utilisée pour l'étalonnage.

11. Procédé selon la revendication 10, **caractérisé en ce que** les échantillons d'étalonnage textiles sont coulés en matières durcissantes, par exemple des polymères, ou des polymères de départ des correspondantes matières fibreuses, par exemple des fontes de viscose, sont utilisées.

12. Procédé selon la revendication 10 ou 11, **caractérisé en ce que** des échantillons d'étalonnage textiles sont utilisés, qui présentent autant que possible la même constante diélectrique que la matière de ruban de fibres à traiter.
